# EUROPEAN PATENT SPECIFICATION

(11) **EP 1 572 333 B1**
(45) Date of publication and mention of the grant of the patent: **05.03.2008**
(21) Application number: 03813097.7
(22) Date of filing: 19.11.2003
(51) Int. Cl.: B01F 3/04, A45D 44/00, B01F 13/10

(54) **TWO STAGE MIXING PROCESS FOR PERSONAL CARE PRODUCTS**
ZWEISTUFIGES MISCHVERFAHREN FÜR KÖRPERPFLEGEMITTEL
PROCEDE DE MELANGE EN DEUX ETAPES POUR DES PRODUITS DE SOIN PERSONNEL

(30) Priority: 16.12.2002 US 320028
(43) Date of publication of application: 14.09.2005
(73) Proprietor: UNILEVER PLC, London EC4P 4BQ (GB); UNILEVER N.V., 3013 AL Rotterdam (NL)
(72) Inventor: BIERCEVICZ, Walter, Anthony, Prospect, CT 06712 (US); MANZARI, Kenneth, Paul, 40 Merritt Boulevard Trumbull, CT 06611 (US); DIVONE, Peter, Anthony, Sr., 40 Merritt Boulevard Trumbull, CT 06611 (US); PRIEST, Kimberly, Ann, Conecticut 06517 (US); REGAN, Joseph, James, 40 Merritt Boulevard Trumbull, CT 06611 (US); JUNGBLUT, Matthew, Rolling Meadows/Chicago Illinois 60008 (US)
(74) Representative: Acham, Nicholas Clive
(86) International application number: PCT/EP2003/013020
(87) International publication number: WO 2004/054693

(56) References cited:
- US-A- 4 272 824
- US-A- 4 628 974
- US-A- 4 835 701
- US-A- 5 163 010

## Description

The invention relates to a process for the manufacture of personal care products wherein variant defining ingredients such as colors, fragrances and/or promotionals are united with a common base formula to efficiently formulate a family of related finished products.

Liquid personal care products of the oil and water emulsion or of the thickened fully aqueous type have traditionally been prepared by batch processes. In these methods, component phases are separately prepared. Then they are combined in a reactor with continuous stirring and heat applied. Quite often separately prepared further phases are added into a main one or two phases. These further phases can either be oily or aqueous, and normally contain product variant defining additives.

Illustrative is US-A-4 272 824 (Lewinger et al.) which discloses a process for batch product preparation. Therein a batch of certain of the product ingredients is mixed in a receptacle, sequentially divided into substantially equal portions and transported to an apparatus for combination with certain other of the product ingredients. US-A-5 163 010 (Klein et al.) discloses a device for formulating a custom mixed cosmetic product at the point of sale in response to specific input criteria.

When a different personal care product variant must be manufactured, the reactor and usually most of its feed lines must be cleaned. Turnaround preparation is costly in both time and material. It is particularly irksome when the laborious turnaround is meant only to produce a slight variant of the first product. Often only a fragrance or color change is necessary in formulating the second product.

A system was sought which would overcome the foregoing problems. More particularly, a system was sought which required less capital in respect to reactors, pumps and piping. Also desirable was more compounding flexibility and faster variant turnaround times. Capability of any new process should be for smaller and shorter runs. Shorter clean-up times between similar variants would be a great advantage. Furthermore, a system was sought having significant material waste reduction and smaller effluent volumes than those of present batch systems.

In a first aspect, there is provided a process for manufacture of personal care compositions which includes:
(i) forming a first aqueous phase portion of a personal care base composition in a first vessel;
(ii) optionally forming a second phase portion of the personal care base composition in a second vessel;
(iii) feeding the first aqueous phase into a blending tube which forms part of a homogenizer, the first aqueous phase moving through the blending tube at a rate from about 2.27 to about 2,270 kg (5 to about 5,000 lbs.) per minute under a pressure ranging from 68.94 kPa to 34.47 MPa (10 to 5,000 psi);
(iv) optionally feeding when present the second phase into the blending tube, the second phase moving through the blending tube at a rate from about 2.27 to about 2,270 kg (5 to about 5,000 lbs.) per minute;
(v) mixing the first aqueous phase and, when present, the second phase within the blending tube;
(vi) downstream from the homogenizer feeding in a continuous manner a late variant addition phase into the mixed phases of the base composition, the late variant addition phase comprising a material selected from the group consisting of a fragrance, a colorant, a promotional ingredient and mixtures thereof, to form a resultant personal care composition; and
(vii) recovering the resultant personal care composition.

Advantages and features of the present invention will become more readily apparent from consideration of the drawings, in which:
Fig. 1 is a schematic flow diagram of a first embodiment of the new process;
Fig. 2 is a schematic flow diagram of a second embodiment of the new process;
Fig. 3 is a schematic flow diagram of a third embodiment of the new process; and
Fig. 4 is a schematic flow diagram of a fourth embodiment of the new process.

Now there has been discovered a late variant addition process for manufacturing a family of ingredient related products. The process first requires preparation of a common base composition. Thereafter, different colorants, fragrance and/or promotional ingredients are blended into the base. Blending of the variant occurs within a small space of tubing and in a continuous flow manner. The variant chemicals are introduced downstream from homogenization, preferably after sonic mixing of the base composition.

The process is intended to protect temperature and shear sensitive additives such as fragrances and organic colorants. Oil and water or solely aqueous phases can be rapidly mixed at relatively high temperatures, while downstream a late variant addition fragrance and/or color composition can be injected at a much lower temperature. Sensitive promotional ingredients, defined herein as materials normally formulated at less than 1 % of the total personal care composition, may also be late variant dosed.

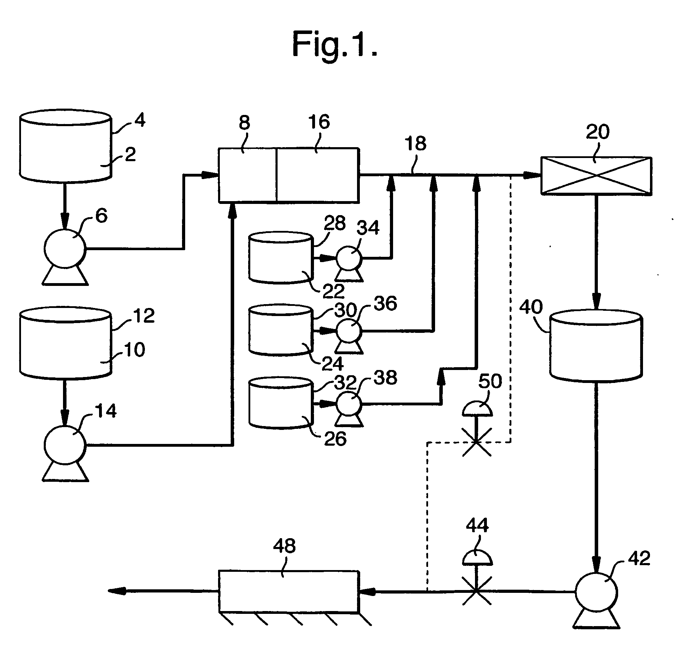

Fig. 1 provides a broad overview of a first embodiment according to the process of this invention. A first aqueous phase 2 is formulated within a tank 4. This phase is delivered via a pump 6 through piping to a blending tube 8. A second phase 10 is held within tank 12. Transfer of the second phase from the tank is achieved by a pump 14 which leads the phase through piping to the blending tube 8.

Often when the intended composition is a skin care product, the formulation will be an emulsion requiring a water and oil phase. In this instance, the second phase will be an oil. Products such as shampoos and shower gels often do not require an oil phase. For these types of products, the second phase will be aqueous and usually deliver one or more surfactants. The levels of surfactants in the shampoos and shower gels generally are well above 10 %. Consequently, these types of products may utilize an aqueous second phase which can include total surfactant levels ranging from about 15 % to about 90 %, preferably from about 25 % to about 75 % by weight of the second phase.

Under some circumstances, the second phase is optional. All but the late variant ingredients can be formulated in the first aqueous phase, which itself serves as the base composition.

Tanks 4 and 12 are fitted with an agitation mechanism such as a paddle stirrer, sonic agitator or pumping system. Also desirable is the presence of a heating mechanism.

Normally the first aqueous phase and second phase may constitute about 75 %, preferably at least about 90 % or more of the total personal care composition. Late variant addition phases will constitute the remainder.

Typically the formulation of the first aqueous phase involves mixing components in tank 4 and maintaining temperature in a range from about 10 to about 70°C, preferably from about 18 to about 58°C, optimally from about 24 to about 52°C. In most but not all instances it will be preferable to have the first aqueous phase at a temperature at least about 5°C, preferably at least about 11°C cooler than the second phase at point of mutual blending.

Subsequent to leaving blending tube 8, the phase or combination of phases 2 and 10 enter a sonolator 16 which operates as a homogenizer thoroughly mixing all phases together under high pressure and shear. The resultant fluid is then transmitted through a conduit 18 into a static mixer 20.

Downstream from the sonolator 16 but prior to entering the static mixer 20, a fluid stream is introduced of colorant phase 22, fragrance phase 24 and promotional ingredient phase 26. Each of the three late variant streams are held in respective vessels 28, 30 and 32. A series of respective pumps 34, 36 and 38 transfer the late variant addition phases into the base composition stream flowing within conduit 18 to the static mixer. The late variant phases are formulated in their respective vessels and transferred to conduit 18 at temperatures ranging from about 0 to about 66°C, preferably from about 10 to about 52°C, optimally from about 24 to about 46°C.

The resultant personal care composition formed in static mixer 20 is transferred to a storage vessel 40. Thereafter the composition can be transported via pump 42 through a back pressure valve 44 into a package filler 48. Rework composition can be moved through the back pressure valve 50 to the static mixer 20.

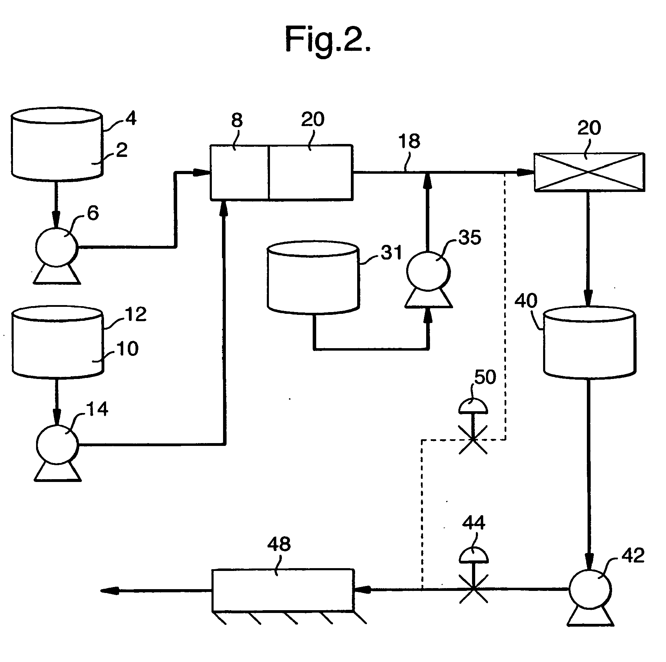

Fig. 2 is a second embodiment very similar to that of the first embodiment. The key difference is the presence of a single late variant phase 31 which contains colorants, fragrances and/or promotional ingredients. The phase is delivered into conduit 18 through use of pump 35.

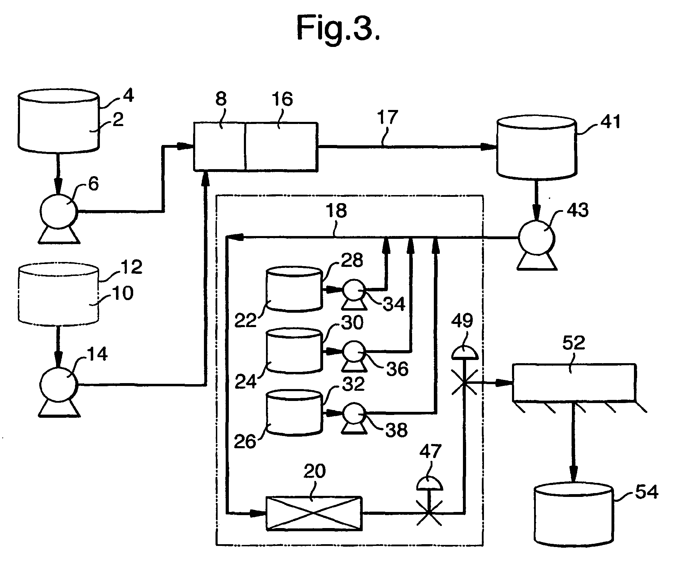

Fig. 3 illustrates a third embodiment of the present invention. Therein, the second phase 10 and tank 12 are shown in phantom as being optional. Instead of immediate transfer to a static mixer, the base composition stream exiting the sonolator 20 is first collected for storage in tank 41 for holding until further formulation into a desired variant. Portions can then be delivered downstream via a pump 43 sending them via conduit 18 to a static mixer 20.

Similar to the process illustrated in Fig. 1, late variant phases for color 22, fragrance 24 and promotional ingredient 26 phases are injected into the conduit by respective pumps 34, 36 and 38. The base composition and the late variant phases are then all blended together within the static mixer 20. A pair of back pressure valves 47 and 49, operating between 5 and 100 psi, regulate transfer of finished personal care composition into a package filling system 52, some of which can also be stored in tank 54. The dashed line box in the figures represents a modular system of tanks, pumps, conduit and mixers which for engineering purposes can be built on a single skid.

For purposes of the present invention, a static mixer is a vessel receiving finished resultant or pre-finished personal care composition and containing a mechanical stirrer serving as primary agitation mechanism against the non-flowing composition. This contrasts with homogenizers (e.g. Sonolator®) which mix primarily by continuous flow of a composition or phase at high rates through an agitation vessel.

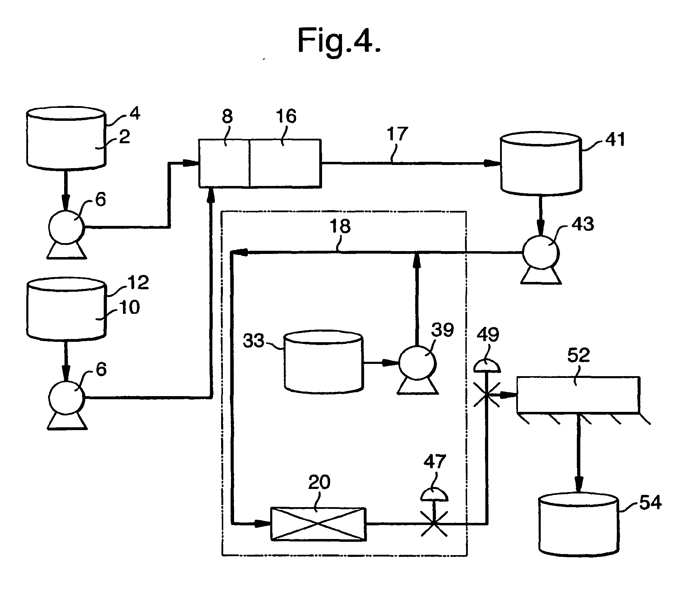

Fig. 4 illustrates a fourth embodiment similar to that of Fig. 3. The primary difference is that the three late variant separate phases are combined into a single phase 33. The latter enters the system via conduit 18 by action of pump 39.

Viscosities of the first aqueous phase may range from about 1 to about 40,000 cps as measured with a Brookfield RVT Viscometer, Spindle No. RV6 at 20 rpm for 1 minute at 25°C.

Preferably the viscosity will range from about 5 to about 500 cps, optimally from about 30 to about 100 cps.

The second phase, particularly when being an oil, can be formed in tank 12 by mixing components at a temperature ranging from about 10 to about 150°C, preferably from about 38 to about 93°C, optimally from about 65 to about 83°C.

Viscosities of the second phase as measured with a Brookfield RVT Viscometer, Spindle No. RV6 at 20 rpm for 1 minute at 25°C, may range from about 50 to about 200,000 cps, preferably from about 1,000 to about 100,000, optimally from 5,000 to about 50,000 cps.

Viscosities of the resultant personal care compositions normally may range between about 1,000 and about 30,000 cps, preferably between about 5,000 and about 30,000 cps, and optimally between 10,000 and 25,000 cps using the Brookfield RVT Viscometer, Spindle No. RV6 at 20 rpm for 1 minute at 25°C.

Delivery of fluids from tanks 4 and 12 into blending tube 8 can be achieved through a pair of electronically controlled servo-driven rotary pumps. Careful control of flow is achieved by use of blending valves available from the Oden Corporation monitored by E&H mass flow meters. The equipment may include static mixing elements and back-pressure valves.

Flow rate for the first and second phases into blending tube 8 may range from about 2.27 to about 2,270 kg (5 to about 5,000 lbs.) per minute. Preferably the flow rate may range from about 22.7 to 454 kg (50 to about 1,000 lbs.) per minute, and optimally from about 68.1 to about 363.2 kg (150 to about 800 lbs.) per minute.

Geometry of the blending tube 8 should be such that residence time of the blended phases may range from about 0.0001 seconds to about 5 minutes, preferably from about 0.001 to about 120 seconds, optimally from about 0.01 to about 10 seconds.

When the second phase is an oil, the temperature of the emulsion in blending tube 8 normally should be less than the temperature of the oil phase as it leaves tank 12. Typical emulsion temperatures within the blending tube 8 may range from about 10 to about 83°C, preferably from about 26 to about 65°C, optimally from about 35 to about 55°C.

In a preferred embodiment, first and second phases are pumped at relatively high pressures which range from 68.94 kPa to 34.47 MPa (10 to 5,000 psi), preferably from 689.4 kPa to 6.894 MPa (100 to 1000 psi), and optimally from 1.034 MPa to 2.068 MPa (150 to 300 psi).

In one embodiment of the present invention, the phases are transferred from their respective tanks 4 and 12 through a positive displacement feed pump such as a Waukesha PD Gear Pump or gravity fed to a triplex pump. Thereafter each of the separate phases are delivered through a high pressure pump such as a triplex plunger type available from the Giant Corporation, Toledo, Ohio or from the Cat Corporation. From there, each of the separate phases are fed into the blending tube 8 which in a preferred embodiment is an antechamber to a Sonolator^{®} available from the Sonic Corporation, unit of General Signal. The Sonolator is an in-line device capable of converting the kinetic energy of a high velocity stream of liquid into a high intensity mixing action. Conversion is accomplished by pumping the liquid through an orifice against a blade-like obstacle immediately in the jet stream of the liquid. The liquid itself oscillates in a stable vortexing pattern, which in turn causes the blade-like obstacle to resonate, resulting in a high level of cavitation, turbulence and shear. The blade or knife is brought into an ultrasonic vibration by the fluid motion which causes cavitation in the liquid.

Alternative high-pressure fed homogenizers other than the Sonolator are the Manton Gaulin type homogenizer available from the APV Manton Corporation and the Microfluidizer available from Microfluidics Corporation. These type high pressure homogenizers contain a valve which is pressed (hydraulically or by a spring) against a fixed valve seat. Under high pressure, fluid flows through the opening in the seat, and then through a gap between the valve and seat. Although geometries of different high pressure homogenizers may differ in details, and may even be roughened with sharp edges, they all are generally similar. Often the high pressure homogenizer may consist of two or more valve-seat combinations.

Personal care compositions according to this invention may include shampoos, shower gels, liquid hand cleansers, liquid dental compositions, skin lotions and creams, hair colorants, facial cleansers, and fluids intended for impregnation onto wiping articles.

Late variant addition phases may be one or more in number. Sometimes these phases may range from about two to about eight in number. They may either be aqueous or oily phases. Fragrances and colorants and promotional ingredients are particularly suitable for a late variant addition because of their temperature sensitivity.

Colorants illustrative of the present invention include Red No. 4, Red No. 40 and the FD&C colorants Red No. 3, Red No. 6, Red No. 28, Red No. 33, Blue No. 1, Green No. 5, Yellow No. 5, all the foregoing being water soluble. Oil soluble dyes may also be utilized such as Green No. 6 and D&C Violet No. 2. Active levels of these colorants may range from about 0.0001 % to about 1 %, preferably from about 0.001 % to about 0.1 % by weight of the total personal care composition.

The term "fragrance" is defined as a mixture of odoriferous components, optionally mixed with a suitable solvent, diluent or carrier, which is employed to impart a desired odor.

Fragrance components and mixtures thereof may be obtained from natural products such as essential oils, absolutes, resinoids, resins and concretes, as well as synthetic products such as hydrocarbons, alcohols, aldehydes, ketones, ethers, carboxylic acids, esters, acetals, ketals, nitriles and the like, including saturated and unsaturated compounds, aliphatic, carbocyclic and heterocyclic compounds.

Typical fragrance ingredients which may be employed for the present invention can be selected from one or more of:
2-Methoxy naphthalene
Allyl cyclohexane propionate
alpha-citronellal
alpha-Ionone
alpha-Santalol
alpha-Terpineol
Ambrettolide
Amyl benzoate
Amyl cinnamate
Amyl cinnamic aldehyde
Aurantiol
Benzaldehyde
Benzophenone
Benzyl acetate
Benzyl salicylate
Beta-caryophyllene
beta-Methyl naphthyl ketone
Cadinene
Cavacrol
Cedrol
Cedryl acetate
Cedryl formate
Cinnamyl cinnamate
cis-Jasmone
Coumarin
Cyclamen aldehyde
Cyclohexyl salicylate
d-Limonene
delta-Nonalactone
delta-Undecalactone
Dihydro isojasmonate
Dihydro mycenol
Dimethyl acetal
Diphenyl methane
Diphenyl oxide
Dodecalactone
Ethyl methyl phenyl glycidate
Ethyl undecylenate
Ethylene brassylate
Eugenol
Exaltolide
Galaxolide
gamma-n-methyl ionone
gamma-Undecalactone
Geraniol
Geranyl acetate
Geranyl anthranilate
Geranyl phenyl acetate
Hexadecanolide
Hexenyl salicylate
Hexyl cinnamic aldehyde
Hexyl salicylate
Hydroxycitronellal
Indole
Iso E super
Iso-Amyl salicylate
Iso-Bornyl acetate
Iso-Butyl quinoline
Iso-Eugenol
Laevo-Carvone
Lilial (p-t-bucinal)
Linalool
Linalyl acetate
Linalyl benzoate
Methyl cinnamate
Methyl dihydrojasmonate
Methyl-N-methyl anthranilate
Musk indanone
Musk ketone
Musk tibetine
Myristicin
Nerol
Oxahexadecanolide-10
Oxahexadecanolide-11
para-Cymene
para-tert-Butyl cyclohexyl acetate
Patchouli alcohol
Phantolide
Phenyl ethyl alcohol
Phenyl ethyl benzoate
Phenyl heptanol
Phenylhexanol
Phenylethylphenylacetate
Thibetolide
Vanillin
Vertenex
Vetiveryl acetate
Yara-yara
Ylangene

Suitable solvents, diluents or carriers for fragrance ingredients as mentioned above are for example ethanol, isopropanol, diethylene glycol monoethyl ether, dipropyl glycol and triethyl citrate.

Particularly preferred fragrance ingredients are cyclic and acyclic terpenes and terpenoids. These materials are based upon isoprene repeating units. Examples include alpha and beta pinene, myrcene, geranyl alcohol and acetate, camphene, dl-limonene, alpha and beta phellandrene, tricyclene, terpinolene, allocimmane, geraniol, nerol, linanool, dihydrolinanool, citral, ionone, methyl ionone, citronellol, citronellal, alpha terpineol, beta terpineol, alpha fenchol, borneol, isoborneol, camphor, terpinen-1-ol, terpin-4-ol, dihydroterpineol, methyl chavicol, anethole, 1,4 and 1,8 cineole, geranyl nitrile, isobornyl acetate, linalyl acetate, caryophyllene, alpha cedrene, guaiol, patchouli alcohol, alpha and beta santalol and mixtures thereof.

Amounts of the fragrance may range from about 0.00001 % to about 2 %, preferably from about 0.0001 % to about 1 %, optimally from about 0.01 % to about 0.5 %, most preferably from about 0.05 % to about 0.25 % by weight of the personal care composition.

Vitamins are illustrative of promotional ingredients added as a late variant. These include Vitamin A (retinol), Vitamin A derivatives (retinyl palmitate, retinyl linoleate, retinyl acetate and retinoic acid), Vitamin C, Vitamin C derivatives (such as ascorbyl tetraisopalmitate and magnesium ascorbyl phosphate), Vitamin E (such as tocopherol acetate), biotin, niacin and DL-panthenol and combinations thereof.

Another popular promotional ingredient is plant extracts. Typical plants from which extracts and other derivatives can be obtained for use in the present invention include the following:
- Wormwood: (Artemisia Absinthium)
- Acacia: (Robinia pseudoacacia)
- Agrimony: (Agrimonia Eupatoria)
- Amryllis: (Amaryllis)
- Colombine: (Aquilegia vulgaris)
- Anemone: (Anemone spp)
- Mugwort: (Artemisia vulgaris)
- Arnica: (Arnica montana)
- Sweet Woodruff: (Asperula odorata)
- Hawthorn: (Crotaegus oxyacantha)
- Azalea: (Azalea spp)
- Balsamine: (Impatiens spp)
- Begonia: (Begonia spp)
- Bougainvillea: (Bougainvillea spp)
- Waterelder: (Viburnum opulus)
- Cornflower: (Centaurea Cyanus)
- Mullein: (Verbascum spp)
- Common heather: (Calluna vulgaris)
- Barbary fig: (Opuntia vulgaris)
- Camellia: (Camellia japonica)
- Chamomile: (Anthemis nobilis)
- Campanula: (Campanula spp)
- Large Indian Cress: (Tropeolum majus)
- Safflower: (Carthamus tinctorius) (Catalpa bignomioides)
- Star thistle: (Centaurea calcitrapa)
- Rough Cherry: (Prunus cerasus)
- Honeysuckle: (Lonicera spp)
- Daisy: (Chrysanthemum leucoanthemum)
- Travelle's Joy: (Clematis vitalba)
- Quince: (Cydonia vulgaris)
- Red poppy: (Papaver Rhoeas)
- Colchicum or Meadow Saffron: (Colchicum automnale saffron)
- Cornel tree (or dogwood): (Cornus spp)
- Crocus: (Crocus spp)
- Cyclamen: (Cyclamen spp)
- Dahlia: (Dahlia variabilis)
- Field larkspur: (Delphinium consolida)
- Dulcamara: (Solanum Dulcamara) (Leontopodium Alpinum)
- Dog rose: (Rosa canina)
- Fumitory: (Fumaria officinalis)
- Broom: (Cytisus scoparius)
- Gentian: (Gentiana spp)
- Geranium: (Geranium spp)
- Wallflower: (Cheirantus cheiri)
- Sword-lily: (Gladialus spp)
- Marsh Mallow: (Althaea officinalis) (Gypsophila spp)
- Roselle: (Hibiscus spp)
- Hydrangea: (Hydrangea spp)
- Hops: (Humulus lupulus)
- Live ever: (Helicrysum arenarium)
- Garden balsam: (Impatiens spp)
- Orrice: (Iris spp)
- Hyacinthe: (Hyacynthus spp)
- Jasmine: (Jasminum spp)
- Jonquil: (Narcissus jonquilla)
- Oleander: (Nerium oleander)
- Lavender: (Lavandule officinalis) (Lavatera spp)
- Lilac: (Syringa vulgaris)
- White lily: (Lilium candidum)
- Bindweed: (Conedvalus spp)
- Lupin: (Lupinus albus)
- Magnolia: (Magnolia spp)
- Daisy: (Chrysanthemum leucanthemum)
- Horsechestnut: (Aesculus Hippocastanum)
- Wild chamomile: (Matricaria chamomilla)
- Mallow: (Malva spp)
- Melilot: (Melilotus officinalis)
- Mint: (Mentha spp)
- St John's Wort: (Hypericum perforatum)
- Mimosa: (Mimosa spp)
- Lion's mouth: (Antirrhinum majus)
- Mugget: (Convallaria maialis)
- Myosotis: (Myosotis spp)
- Daffodil: (Narcissus spp)
- White water Lily: (Nymphaea alba)
- Gilower: (Dianthus caryophyllus)
- Marigold: (Tagetes spp)
- Sweet orange Tree Orchid: (Citrus Aurantium)
- Daisy: (Bellis perennis)
- Passion flower: (Passiflora spp)
- Peach-tree: (Prunus persica)
- Pelargonium: (Pelargonium spp)
- Pansy: (Viola spp)
- Snowdrop: (Galanthus nivalis)
- Periwinkle: (Vinca spp)
- Petunia: (Petunia spp)
- Phlox: (Phlox spp)
- Field larkspur: (Delphinium consolida)
- Garden peony: (Paeonia officinalis)
- Sweat pea: (Lathyrus odorantes) (Polygonum spp)
- Apple tree: (Pirus malus)
- Primrose: (Primula spp)
- Silver weed: (Potentilla Anserina)
- Plum-tree: (Prunus domestica)
- Pyrethum: (Chrysanthemum cineriaefolium)
- Meadow Sweet: (Spiraea Ulmaria)
- Buttercup: (Ranuncukus spp)
- Rhododendron: (Rhododendron ferrugineum)
- Rose mary: (Rosmarinus officinalis)
- French Rose: (Rose gallica)
- Saffron: (Crocus sativus)
- Grass polly: (Lythrum salicaria)
- Bloodroot: (Sanguinaria canadiensis)
- Soapwort: (Saponaria officinalis)
- Sage: (Salvia officinalis)
- Willow: (Salix alba)
- Devil's bit scabiou: (Scabiosa Succisa)
- Syringa: (Philadelphus coronarius)
- Serpollet: (Thymus serpylum) (Sophora japonica)
- Corme: (Sorbus domestica)
- Marigold: (Calandula officinalis)
- Spiraea: (Spiraea spp)
- Elder: (Sambucus nigra)
- Tamarisk: (Tamaris gallica)
- Tansy: (Tanatecum vulgare)
- Garden thyme: (Thymus vulgaris)
- Lime: (Tilia spp)
- Clover: (Trifolium spp)
- Tulip: (Tulipa spp)
- Coltsfoot: (Tussilago Iarfara)
- Speedwell: (Veronica officinalis)
- Common vervain: (Verbena officinalis)
- Violet: (Viola spp)
- Yucca: (Yuccas spp)

Amounts of the promotional ingredients may range from about 0.00001 % to about 2 %, preferably from about 0.0001 % to about 1 %, optimally from about 0.001 % to about 0.5 % by weight of the total personal care composition.

The first phase may constitute from about 5 % to about 99.5 %, preferably from about 20 % to about 90 %, more preferably from about 35 % to about 80 %, optimally from about 45 % to about 70 % by weight of the final resultant personal care composition.

The first phase will contain water as a major component. Usually the amount of water may range from about 30 % to about 99.9 %, preferably from about 50 % to about 95 %, more preferably from about 65 % to about 80 %, optimally from about 55 % to about 70 % by weight of the water phase.

Generally the first phase will contain a surfactant. Useful surfactants include nonionic, anionic, cationic, amphoteric, zwitterionic and surfactant combinations thereof. Overall amount of surfactant may range from about 0.1 % to about 50 %, preferably from about 2 % to about 40 %, optimally from about 15 % to about 25 % by weight of the total personal care composition.

Illustrative but not limiting examples of suitable nonionic surfactants include C₁₀-C₂₀ fatty alcohol or acid hydrophobes condensed with from 2 to 100 moles of ethylene oxide or propylene oxide per mole of hydrophobe; C₂-C₁₀ alkyl phenols condensed with from 2 to 20 moles of alkylene oxides; mono- and di- fatty acid esters of ethylene glycol such as ethylene glycol distearate; fatty acid monoglycerides; sorbitan mono- and di- C₈-C₂₀ fatty acids; and polyoxyethylene sorbitan available as Polysorbate 80 and Tween 80^{®} as well as combinations of any of the above surfactants.

Other useful nonionic surfactants include alkyl polyglycosides (APGs), saccharide fatty amides (e.g. methyl gluconamides) as well as long chain tertiary amine oxides. Examples of the latter category are: dimethyldodecylamine oxide, oleyldi(2-hydroxyethyl)amine oxide, dimethyloctylamine oxide, dimethyldecylamine oxide, dimethyltetradecylamine oxide, di(2-hydroxyethyl) tetradecylamine oxide, 3-didodecyloxy-2-hydroxypropyldi(3-hydroxypropyl) amine oxide, and dimethylhexadecylamine oxide.

Illustrative but not limiting examples of anionic surfactants include the following:
(1) Alkyl benzene sulfonates in which the alkyl group contains from 9 to 15 carbon atoms, preferably 11 to 14 carbon atoms in straight chain or branched chain configuration. Especially preferred is a linear alkyl benzene sulfonate containing about 12 carbon atoms in the alkyl chain.
(2) Alkyl sulfates obtained by sulfating an alcohol having 8 to 22 carbon atoms, preferably 12 to 16 carbon atoms. The alkyl sulfates have the formula ROSO₃-M⁺ where R is the C₈₋₂₂ alkyl group and M is a mono- and/or divalent cation.
(3) Paraffin sulfonates having 8 to 22 carbon atoms, preferably 12 to 16 carbon atoms, in the alkyl moiety.
(4) Olefin sulfonates having 8 to 22 carbon atoms, preferably 12 to 16 carbon atoms. Most preferred is sodium C₁₄-C₁₆ olefin sulfonate, available as Bioterge AS 40^{®}.
(5) Alkyl ether sulfates derived from an alcohol having 8 to 22 carbon atoms, preferably 12 to 16 carbon atoms, ethoxylated with less than 30, preferably less than 12, moles of ethylene oxide. Most preferred is sodium lauryl ether sulfate formed from 2 moles average ethoxylation, commercially available as Standopol ES-2^{®}.
(6) Alkyl glyceryl ether sulfonates having 8 to 22 carbon atoms, preferably 12 to 16 carbon atoms, in the alkyl moiety.
(7) Fatty acid ester sulfonates of the formula: R¹CH(SO₃-M+)CO2R² where R¹ is straight or branched alkyl from about C₈ to C₁₈, preferably C₁₂ to C₁₆, and R² is straight or branched alkyl from about C₁ to C₆, preferably primarily C₁, and M+ represents a mono- or divalent cation.
(8) Secondary alcohol sulfates having 6 to 18, preferably 8 to 16 carbon atoms.
(9) Fatty acyl isethionates having from 10 to 22 carbon atoms, with sodium cocoyl isethionate being preferred.
(10) Mono- and dialkyl sulfosuccinates wherein the alkyl groups range from 3 to 20 carbon atoms each.
(11) Alkanoyl sarcosinates corresponding to the formula RCON(CH₃)CH₂CH₂CO₂M wherein R is alkyl or alkenyl of about 10 to about 20 carbon atoms and M is a watersoluble cation such as ammonium, sodium, potassium and trialkanolammonium. Most preferred is sodium lauroyl sarcosinate.

Illustrative of cationic surfactants are C₈-C₂₂ alkyl C₁-C₄ di-alkyl ammonium salts such as cetyl dimethyl ammonium chloride, stearyl dimethyl ammonium methosulfate, oleyl diethylammonium phosphate, and lauryl dimethyl ammonium borate. Particularly preferred is cetrimonium chloride which is a generic term for cetyl dimethyl ammonium chloride.

Amphoteric surfactants useful for the present invention include betaines which may have the general formula RN⁺(R¹)₂R²-COO⁻ wherein R is a hydrophobic moiety selected from the group consisting of alkyl groups containing from 10 to 22 carbon atoms, preferably from 12 to 18 carbon atoms; alkyl aryl and aryl alkyl groups containing 10 to 22 carbon atoms with a benzene ring being treated as equivalent to about 2 carbon atoms, and similar structures interrupted by amido or ether linkages; each R¹ is an alkyl group containing from 1 to 3 carbon atoms; and R² is an alkylene group containing from 1 to about 6 carbon atoms. Sulfobetaines such as cocoamidopropyl hydroxysultaine are also suitable.

Examples of preferred betaines are dodecyl dimethyl betaine, cetyl dimethyl betaine, dodecyl amidopropyldimethyl betaine, tetradecyldimethyl betaine, tetradecylamidopropyldimethyl betaine, and dodecyldimethylammonium hexanoate. Most preferred is cocoamidopropyl betaine available as Tegobetaine F^{®} sold by Th. Goldschmidt AG of Germany.

Polyols are frequently present in the first phase of the present invention. Typical polyhydric alcohols include glycerol (also known as glycerin), polyalkylene glycols and more preferably alkylene polyols and their derivatives, including propylene glycol, dipropylene glycol, polypropylene glycol, polyethylene glycol and derivatives thereof, sorbitol, hydroxypropyl sorbitol, hexylene glycol, butylene glycol, 1,2,5-hexanetriol, ethoxylated glycerol, propoxylated glycerol and mixtures thereof. Most preferred is glycerin. Amounts of the polyols may range from about 0.5 % to about 50 %, preferably between about 1 % and about 15 % by weight of the total personal care composition.

Thickeners/viscosifiers in amounts from about 0.01 % to about 10 % by weight of the total personal care composition may also be included in the first phase. As known to those skilled in the art, the precise amount of thickeners can vary depending upon the consistency and thickness of the composition which is desired. Exemplary thickeners are xanthan gum, sodium carboxymethyl cellulose, hydroxyalkyl and alkyl celluloses (particularly hydroxypropyl cellulose), and cross-linked acrylic acid polymers such as those sold by B.F. Goodrich under the Carbopol trademark. Thickeners such as modified starches and clays may also be used to thicken the water phase. For instance, aluminum starch octenyl succinate (available as DryFlo^{®} from the National Starch and Chemical Company) is particularly useful. Among the clays are included magnesium aluminum silicate (available as Veegum^{®}), hectorite clays, montmorillonite clays, bentonites (e.g. Bentone^{®} 38) and combinations thereof.

Water soluble conditioning agents may also be incorporated into the first phase. Cationic agents in monomeric and polymeric form are particularly useful for this purpose. Cationic cellulose derivatives, cationic starches, copolymers of a diallyl quaternary ammonium salt and an acrylamide, quaternized vinylpyrrolidone vinylimidazole polymers, polyglycol amine condensates, quaternized collagen polypeptide, polyethylene imine, cationized silicone polymer (e.g. Amodimethicone), cationic silicone polymers providied in a mixture with other components under the trademark Dow Corning 929 (cationized emulsion), copolymers of adipic acid and dimethylaminohydroxypropyl diethylenetriamine, cationic chitin derivatives, cationized guar gum (e.g. Jaguar^{®} C-B-S, Jaguar^{®} C-17, and Jaguar^{®} C-16, quaternary ammonium salt polymers (e.g. Mirapol^{®} A-15, Mirapol^{®} AD-1, Mirapol^{®}, ZA-1, etc., manufactured by the Miranol Division of the Rhone Poulenc Company). Examples of the monomeric cationic conditioning agents are salts of the general structure: 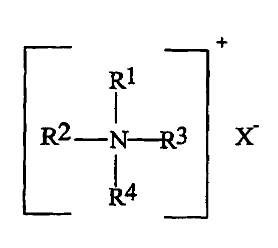 wherein R¹ is selected from an alkyl group having from 12 to 22 carbon atoms, or aromatic, aryl or alkaryl groups having from 12 to 22 carbon atoms; R², R³, and R⁴ are independently selected from hydrogen, an alkyl group having from 1 to 22 carbon atoms, or aromatic, aryl or alkaryl groups having from 12 to 22 carbon atoms; and X is an anion selected from chloride, bromide, iodide, acetate, nitrate, sulfate, methyl sulfate, ethyl sulfate, tosylate, lactylate, citrate, glycolate, and mixtures thereof. Additionally, the alkyl groups can also contain either linkages or amino group substituents (e.g., the alkyl groups can contain polyethylene glycol and polypropylene glycol moieties).

Amounts of each cationic conditioning agent may range from about 0.05 % to about 5 %, preferably from about 0.1 % to about 3 %, optimally from about 0.3 % to about 2.5 % by weight of the total personal care composition.

Compositions of the present invention can be water and oil emulsions. They may be oil-in-water or water-in-oil, although the former is preferred. Relative weight ratios of water to oil representing first and second phases may range from about 1,000:1 to about 1:10, preferably from about 100:1 to about 1:5, optimally from about 10:1 to about 1:2 by weight of the total personal care composition.

Another component often present in the first phase are preservatives. These are incorporated to protect against the growth of potentially harmful microorganism. Suitable traditional preservatives are EDTA salts and alkyl ester of parahydroxybenzoic acid. Other preservatives which have more recently come into use include hydantoin derivatives, propionate salts, and a variety of quaternary ammonium compounds. Cosmetic chemists are familiar with appropriate preservatives and routinely choose them to satisfy the preservative challenge test and to provide product stability. Particularly preferred preservatives are iodopropynyl butyl carbamate, phenoxyethanol, methyl paraben, propyl paraben, imidazolidinyl urea, sodium dehydroacetate and benzyl alcohol. The preservatives should be selected having regard for the use of the composition and possible incompatabilities between the preservatives and other ingredients in the composition. Preservatives are employed in amounts ranging from 0.01 % to 2 % by weight of the total personal care composition.

When present, the second (oil) phase may contain hydrophobic components. Most often the oil phase will incorporate an emollient which may be selected from hydrocarbons, silicones and synthetic or vegetable esters. Amounts of the emollients may range anywhere from about 0.1 % to about 30 %, preferably between about 0.5 % and about 10 % by weight of the total personal care composition.

Hydrocarbons suitable for the present invention include isoparaffins, mineral oil, petrolatum and hydrocarbon waxes such as polyethylenes.

Silicones may be divided into the volatile and non-volatile variety. The term "volatile" as used herein refers to those materials which have a measurable vapor pressure at ambient temperature. Volatile silicone oils are preferably chosen from cyclic or linear polydimethylsiloxanes containing from about 3 to about 9, preferably from about 4 to about 5, silicon atoms.

Nonvolatile silicones useful as an emollient material include polyalkyl siloxanes, polyalkylaryl siloxanes and polyether siloxane copolymers. The essentially non-volatile polyalkyl siloxanes useful herein include, for example, polydimethyl siloxanes with viscosities of from about 5 to about 100,000 centistokes at 25°C.

Among suitable ester emollients are:
(1) Alkenyl or alkyl esters of fatty acids having 10 to 20 carbon atoms. Examples thereof include isopropyl palmitate, isononyl isononoate, oleyl myristate, oleyl stearate, cetearyl stearate and oleyl oleate.
(2) Ether-esters such as fatty acid esters of ethoxylated fatty alcohols.
(3) Polyhydric alcohol esters. Ethylene glycol mono- and di-fatty acid esters, diethylene glycol mono- and di-fatty acid esters, polyethylene glycol (200-6000) mono- and di-fatty acid esters, propylene glycol mono- and di-fatty acid esters, polypropylene glycol 2000 monooleate, polypropylene glycol 2000 monostearate, ethoxylated propylene glycol monostearate, glyceryl mono- and di-fatty acid esters, polyglycerol fatty esters, ethoxylated glyceryl monostearate, 1,3-butylene glycol monostearate, 1,3-butylene glycol distearate, polyoxyethylene polyol fatty acid ester, sorbitan fatty acid esters, and polyoxyethylene sorbitan fatty acid esters are satisfactory polyhydric alcohol esters.
(4) Wax esters such as beeswax, spermaceti, myristyl myristate, stearyl stearate.
(5) Steroid esters, of which soya sterol and cholesterol fatty acid esters are examples thereof.

Most preferred vegetable ester emollients are sunflower seed oil, soy sterol esters, borage seed oil, maleated soybean oil, sucrose polycottonseedate, tribehenin, sucrose polybehenate and mixtures thereof.

Fatty acids may also be included in the oil phase. These fatty acids may have from 10 to 30 carbon atoms. Illustrative of this category are pelargonic, lauric, myristic, palmitic, stearic, isostearic, hydroxystearic, oleic, linoleic, ricinoleic, arachidic, behenic and erucic acids. Amounts may range from 0.1 % to 25 % by weight of the total personal care composition.

The term "comprising" is meant not to be limiting to any subsequently stated elements but rather to encompass non-specified elements of major or minor functional importance. In other words the listed steps, elements or options need not be exhaustive. Whenever the words "including" or "having" are used, these terms are meant to be equivalent to "comprising" as defined above.

### EXAMPLES

Except in the operating and comparative examples, or where otherwise explicitly indicated, all numbers in this description indicating amounts of material ought to be understood as modified by the word "about".

The following examples will more fully illustrate the embodiments of this invention. All parts, percentages and proportions referred to herein and in the appended claims are by weight unless otherwise illustrated.

### Example 1

### Skin Lotion

A skin lotion is prepared according to the following procedure. In a first tank, a water phase is formulated. Components are shown in Table IA. Temperature is maintained between 24° and 46°C.

**TABLE IA**

| Water Phase | |
|---|---|
| INGREDIENT | WEIGHT % |
| Glycerin | 7.00 |
| Carbopol 934® (2% Active) | 3.00 |
| Triethanolamine | 0.80 |
| Magnesium Aluminum Silicate | 0.40 |
| Glydant Plus® | 0.15 |
| Disodium EDTA | 0.10 |
| Water | 72.28 |
| Total | 83.73 |

Separately an oil phase is prepared in a second tank. Components of that phase are outlined in Table IB below. The temperature of the oil phase is maintained between 65° and 88°C. A pair of progressive cavity pumps (ex Moyno/Seepex) are utilized to transport the oil and water phases from their respective tanks at rates of approximately 51.30 and 380.0 kg (113 and 837 lbs.) per minute, respectively. These phases are delivered through pipes which join eventually leading into a blending tube which is an antechamber section of a Sonolator^{®}.

**TABLE IB**

| Oil Phase | |
|---|---|
| INGREDIENT | WEIGHT % |
| Stearic Acid | 4.00 |
| Glycol Stearate/Stearamide AMP | 2.00 |
| Glycerol Monostearate | 0.80 |
| Cetyl Alcohol | 0.60 |
| Sunflower Seed Oil | 1.40 |
| Petrolatum | 1.30 |
| Methyl Palmitate | 0.50 |
| Dimethicone | 0.32 |
| Sodium Stearoyl Lactylate | 0.20 |
| Soya Sterol | 0.10 |
| Vitamin E Acetate | 0.01 |
| Lecithin | 0.04 |
| Total | 11.27 |

The resultant blend is then homogenized as liquid flows through an orifice against the blade-like obstacle immediately in the jetstream of the liquid. This liquid is subject to ultrasonic vibration causing cavitation in the liquid. Sonolation pressure is held at approximately 3,000 psi. From the Sonolator^{®}, the liquid which represents 95 % of the final skin lotion receives a late variant addition phase stream representing the remaining 5 % of the resultant skin lotion. Introduction of the late variant addition phase stream occurs along a flow path of liquids moving through conduit piping leading to a static mixer. All streams are then further blended in the static mixer (with back pressure maintained at 20 psi). The late variant addition phase components are outlined in Table I(C). Temperature of the late variant addition phase is maintained between 24° and 46°C.

**TABLE IC**

| Late Variant Addition Phase | |
|---|---|
| INGREDIENT | WEIGHT % |
| Water | 4.499 |
| Titanium Dioxide | 0.10 |
| Fragrance | 0.15 |
| Colorant | 0.25 |
| Vitamin A Palmitate | 0.001 |
| Total | 5.00 |

Subsequent to treatment in the static mixer, the mixed skin lotion is transferred to a storage vessel. Individual bottles are filled on a package line fed from the storage vessel.

### Example 2

### Shampoo

A shampoo is prepared according to the following procedure. In a tank, a water phase is formulated. Components are shown in Table IIA. Temperature is maintained between 24° and 46°C.

**TABLE IIA**

| Water Phase | |
|---|---|
| Ingredient | Weight % |
| Water | 75.00 |
| Almeo Blend* | 18.00 |
| Citric Acid (50% in Water) | 0.02 |
| Hydroxypropylmethylcellulose | 0.15 |
| Tetrasodium EDTA | 0.20 |
| Glydant® | 0.13 |
| Kathon CG® | 0.04 |
| Benzophenone-4 | 0.05 |
| Ammonium Chloride | 1.40 |

| | |
|---|---|
| * Includes: 22.85% Sodium Lauryl Sulfate; 29.25% Sodium Lauryl Ether Sulfate; 5% Monoethanolamine; 2.5% PEG-5; citric acid and preservative. | |

The water phase is transferred at 43.13 kg (95 lbs.) per minute via a triplex cat pump to the blending tube section of a Sonolator^{®}. Pressure within the Sonolator^{®} is held at 800 psi. After homogenization, the water phase is transported through a conduit leading to a static mixer. A late variant addition phase is interjected at 2.27 kg (5 lbs.) per minute into the conduit thereby combining with the homogenized water phase. The late variant addition phase consists of the components as listed in Table IIB.

**TABLE IIB**

| Late Variant Addition Phase | |
|---|---|
| Ingredient | Weight % |
| Water | 4.50774 |
| Fragrance | 0.50 |
| D&C Red #33 | 0.00024 |
| FD&C Blue #1 | 0.00002 |
| Vitamin E Acetate | 0.001 |
| Herbal Extract | 0.001 |

The combined water phase and late variant addition phases are then agitated in the static mixer (controlled by a back pressure valve at 20 psi). Thereafter, the resultant shampoo composition is fed to a storage vessel. Empty bottles on a packaging line are filled by pumping the shampoo composition from the storage vessel via a positive displacement pump into filler conduits delivering product into the empty bottles.

### Example 3

### Hair Conditioner

A hair conditioner is prepared according to the following procedure. In a first tank, a water phase is formulated. Components are shown in Table IIIA. Temperature is maintained between 24° and 46°C.

**TABLE IIIA**

| Water Phase | |
|---|---|
| Ingredient | Weight % |
| Water | 46.785 |
| Potassium Chloride | 0.30 |
| Disodium EDTA | 0.10 |
| Kathon CG® | 0.05 |
| 2-Bromo-2-Nitropropanot-1 | 0.03 |

Separately an oil (emulsion type) phase is prepared in a second tank. Components of that phase are outlined in Table IIIB below. Temperature of the oil phase is maintained between 65° and 88°C.

**TABLE IIIB**

| Oil (Emulsion) Phase | |
|---|---|
| Ingredient | Weight % |
| Water | 41.785 |
| Distearyl Dimonium Chloride | 0.15 |
| Cetrimonium Chloride (30% Active) | 2.80 |
| Cetyl Alcohol | 3.00 |

A pair of progressive cavity pumps (ex Moyno/Seepex) are utilized to transport the oil and water phases from their respective tanks at rates of approximately 216.56 and 214.74 kg (477 lbs. and 473 Ibs.) per minute, respectively. These phases are delivered through pipes which join eventually leading into a blending tube which is an antechamber section of a Sonolator®.

The resultant blend is then homogenized as liquid flows through an orifice against the blade-like obstacle immediately in the jetstream of the liquid. This liquid is subject to ultrasonic vibration causing cavitation in the liquid. Sonolation pressure is held at approximately 3,000 psi. From the Sonolator^{®}, the liquid which represents 95 % of the final hair conditioner receives a late variant addition phase stream representing the remaining 5% of the resultant hair conditioner. Introduction of these streams occurs along a flow path of liquids moving through conduit piping leading to a static mixer. All streams are then further blended in the static mixer (with back pressure maintained at 20 psi). The late variant addition phase components are outlined in Table III(C). Temperature of the late variant addition phase is maintained between 24° and 46°C.

**TABLE IIIC**

| Late Variant Addition Phase | |
|---|---|
| Ingredient | Weight % |
| Water | 4.79788 |
| Fragrance | 0.20 |
| Vitamin E Acetate | 0.001 |
| Herbal Extract | 0.001 |
| D&C Red #33 | 0.0001 |
| D&C Orange #4 | 0.00002 |

Subsequent to treatment in the static mixer, the mixed hair conditioner is transferred to a storage vessel. Individual bottles are filled on a package line fed from the storage tank.

### Example 4

### Body Wash

A body wash is prepared according to the following procedure. In a tank, a water phase is formulated. Components are shown in Table IVA. Temperature is maintained between 24° and 46°C.

**TABLE IVA**

| Water Phase | |
|---|---|
| Ingredient | Weight % |
| Water | 69.953 |
| Almeo Blend | 17.370 |
| Cocoamidopropyl Betaine | 6.028 |
| Aloe Vera Powder | 0.005 |
| Benzophenone-4 | 0.100 |
| Glycerin | 1.000 |
| PEG-10 Sunflower Glyceride | 0.100 |
| Tetrasodium EDTA | 0.051 |
| Sodium Hydroxide (50% Aqueous) | 0.048 |
| Ammonium Chloride | 0.325 |
| Kathon CG® | 0.020 |

The water phase is transferred at 431.3 kg (950 lbs.) per minute via a triplex cat pump to the blending tube section of a Sonolator^{®}. Pressure within the Sonolator^{®} is held at 1000 psi. After homogenization, the water phase is transported through a conduit leading to a static mixer. A late variant addition phase is interjected at 22.7 kg (50 lbs.) per minute into the conduit thereby combining with the homogenized water phase. The late variant addition phase consists of the components as listed in Table IVB.

**TABLE IVB**

| Late Variant Addition Phase | |
|---|---|
| Ingredient | Weight % |
| Water | 3.534 |
| Fragrance | 1.000 |
| Violet #2 (0.2% Active) | 0.333 |
| Dequest 2010® | 0.033 |
| Polyquaternium-10 | 0.100 |

The combined water and late variant addition phases are then agitated in the static mixer (controlled by a back pressure valve at 20 psi). Thereafter, the resultant body wash composition is fed to a storage vessel. Empty bottles on a packaging line are filled by pumping the body wash composition from the storage vessel via a positive displacement pump into filler conduits delivering product into the empty bottles.

## Claims

1. A process for manufacture of a personal care composition comprising:
(i) forming a first aqueous phase portion of a personal care base composition in a first vessel;
(ii) optionally forming a second phase portion of the personal care base composition in a second vessel;
**characterized by**
(iii) feeding the first aqueous phase into a blending tube which forms part of a homogenizer, the first aqueous phase moving through the blending tube at a rate from 2.27 to 2,270 kg (5 to 5,000 lbs.) per minute under a pressure ranging from 68.94 kPa to 34.47 MPa (10 to 5,000 psi);;
(iv) optionally feeding when present the second phase into the blending tube, the second phase moving through the blending tube at a rate from 2.27 to 2,270 kg (5 to 5,000 lbs.) per minute;
(v) mixing the first aqueous phase and, when present, the second phase within the blending tube;
(vi) downstream from the homogeniser feeding in a continuous manner a late variant addition phase into the mixed phases of the base composition, the late variant addition phase comprising a material selected from a fragrance, a colorant, a promotional ingredient and mixtures thereof, to form a resultant personal care composition; and
(vii) recovering the resultant personal care composition.

2. The process according to claim 1 wherein the first aqueous phase comprises from 0.1 % to 50 % by weight of the personal care composition of a surfactant.

3. The process according to claim 1 or claim 2 wherein the first and second phases are present in a relative ratio from 1,000:1 to 1:10 by weight of the personal care composition.

4. The process according to any one of the preceding claims wherein the homogenizer delivers sonic agitation to the first phase.

5. The process according to any one of the preceding claims wherein the material of the late variant addition phase is a fragrance.

6. The process according to any one of the preceding claims wherein the material of the late variant addition phase is a colorant.

7. The process according to any one of the preceding claims wherein the material of the late variant addition phase is a promotional ingredient selected from vitamins, plant extract and mixtures thereof.

8. The process according to any one of the preceding claims wherein the second phase is an oil.

## Patentansprüche

1. Verfahren zur Herstellung einer Körperpflegezusammensetzung, umfassend:
(i) Bilden eines ersten wässrigen Phasenteils von einer Körperpflegegrundzusammensetzung in einem ersten Gefäß;
(ii) gegebenenfalls Bilden eines zweiten Phasenteils von der Körperpflegegrundzusammensetzung in einem zweiten Gefäß;
**gekennzeichnet durch**
(iii) Zuführen der ersten wässrigen Phase in ein Mischrohr, das Teil eines Homogenisators bildet, wobei die erste wässrige Phase sich **durch** das Mischrohr mit einer Geschwindigkeit von 2,27 bis 2270 kg (5 bis 5000 lbs) pro Minute unter einem Druck im Bereich von 68,94 kPa bis 34,47 MPa (10 bis 5000 psi) bewegt;
(iv) gegebenenfalls Zuführen, falls vorliegend, der zweiten Phase in das Mischrohr, wobei die zweite Phase sich **durch** das Mischrohr mit einer Geschwindigkeit von 2,27 bis 2270 kg (5 bis 5000 lbs) pro Minute bewegt;
(v) Mischen der ersten wässrigen Phase und, falls vorliegend, der zweiten Phase innerhalb des Mischrohrs;
(vi) stromabwärts von dem Homogenisator Zuführen in einer kontinuierlichen Weise einer Phase von einer Zugabe der späten Variante in die Mischphasen der Grundzusammensetzung, wobei die Phase von einer Zugabe der späten Variante ein Material umfasst, ausgewählt aus einem Duftstoff, einem Färbemittel, einem fördernden Bestandteil und Gemischen davon, um die erhaltene Körperpflegezusammensetzung zu bilden, und
(vii) Gewinnen der erhaltenen Körperpflegezusammensetzung.

2. Verfahren nach Anspruch 1, wobei die erste wässrige Phase 0,1 % bis 50 Gew.-% der Körperpflegezusammensetzung von einem Tensid umfasst.

3. Verfahren nach Anspruch 1 oder Anspruch 2, wobei die ersten und zweiten Phasen mit einem relativen Verhältnis von 1000 : 1 bis 1 : 10, auf das Gewicht der Körperpflegezusammensetzung, vorliegen.

4. Verfahren nach einem der vorangehenden Ansprüche, wobei der Homogenisator Ultraschallbewegung an die erste Phase abgibt.

5. Verfahren nach einem der vorangehenden Ansprüche, wobei das Material der Phase von einer Zugabe der späten Variante ein Duftstoff ist.

6. Verfahren nach einem der vorangehenden Ansprüche, wobei das Material der Phase von einer Zugabe der späten Variante ein Färbemittel ist.

7. Verfahren nach einem der vorangehenden Ansprüche, wobei das Material der Phase von einer Zugabe der späten Variante ein fördernder Bestandteil ist, ausgewählt aus Vitaminen, Pflanzenextrakt und Gemischen davon.

8. Verfahren nach einem der vorangehenden Ansprüche, wobei die zweite Phase ein Öl darstellt.

## Revendications

1. Procédé de fabrication d'une composition de soins personnels comprenant :
(i) la formation d'une première portion de phase aqueuse d'une composition de base de soins personnels dans un premier récipient ;
(ii) la formation facultative d'une seconde portion de phase d'une composition de base de soins personnels dans un second récipient ;
**caractérisé par**
(iii) l'alimentation de la première phase aqueuse dans un tube de mélange faisant partie d'un homogénéisateur, la première phase aqueuse se déplaçant au travers du tube de mélange à une vitesse de 2,27 à 2 270 kg (5 à 5 000 livres) par minute sous une pression allant de 68,94 kPa à 34,47 MPa (10 à 5 000 psi) ;
(iv) l'alimentation facultative lorsqu'elle est présente de la seconde phase dans le tube de mélange, la seconde phase se déplaçant au travers du tube de mélange à une vitesse allant de 2,27 à 2 270 kg (5 à 5 000 livres) par minute ;
(v) le mélange de la première phase aqueuse avec, lorsqu'elle est présente, la seconde phase à l'intérieur du tube de mélange ;
(vi) en aval de l'homogénéisateur, l'alimentation de façon continue d'une dernière phase supplémentaire variable comprenant un matériau choisi parmi un parfum, un colorant, un ingrédient stimulant et des mélanges de ceux-ci, afin de former une composition de soins personnels la récupération de la composition de soins personnels résultante.

2. Procédé selon la revendication 1, dans lequel la première phase aqueuse comprend de 0,1 % à 50 % du poids de la composition de soins personnels d'un agent tensioactif.

3. Procédé selon la revendication 1 ou la revendication 2, dans lequel la première et la seconde phases sont présentes dans des rapports relatifs allant de 1 000:1 à 1:10 du poids de la composition de soins personnels.

4. Procédé selon l'une quelconque des revendications précédentes, dans lequel l'homogénéisateur fournit une agitation sonique à la première phase.

5. Procédé selon l'une quelconque des revendications précédentes, dans lequel le matériau de la phase supplémentaire variable ultérieure est un parfum.

6. Procédé selon l'une quelconque des revendications précédentes, dans lequel le matériau de la phase supplémentaire variable ultérieure est un colorant.

7. Procédé selon l'une quelconque des revendications précédentes, dans lequel le matériau de la phase supplémentaire variable ultérieure est un ingrédient stimulant choisi parmi des vitamines, des extraits de plante et des mélanges de ceux-ci.

8. Procédé selon l'une quelconque des revendications précédentes, dans lequel la seconde phase est une huile.
